# EUROPÄISCHE PATENTANMELDUNG

(11) **EP 0 890 960 A1**
(43) Veröffentlichungstag der Anmeldung: **13.01.1999**
(21) Anmeldenummer: 98250242.9
(22) Anmeldetag: 01.07.1998
(51) Int. Cl.: G21G 4/08

(54) **Radioaktive Strahlenquelle zur Behandlung von Gefässanomalien**

(30) Priorität: 08.07.1997 DE 19729075
(71) Anmelder: Bebig Isotopentechnik und Umweltdiagnostik GmbH, 13125 Berlin (DE)
(72) Erfinder: Ziegler, Jürgen, Dipl.-Ing., 12679 Berlin (DE); Eckert, Andreas, Dr., 16341 Zepernick (DE)
(74) Vertreter: Ziebig, Marlene, Dr. Dipl.-Chem.

(57) **Zusammenfassung**

Die Erfindung betrifft eine radioaktive Strahlenquelle zur Behandlung von Gefäßanomalien, die ein Nuklid oder ein Nuklidgemisch mit 70-90% β-Strahlung und 30-10% γ-Strahlung beinhaltet, vorzugsweise das Nuklid Ruthenium-106. Die Strahlenquelle ist besonders vorteilhaft zur "in stent therapy" einsetzbar.

## Beschreibung

Die Erfindung betrifft eine radioaktive Strahlenquelle zur Behandlung von Gefäßanomalien, die ein Nuklid oder ein Nuklidgemisch mit 70-90% β-Strahlung und 30-10% γ-Strahlung beinhaltet, vorzugsweise das Nuklid Ruthenium-106.

In den Blutgefäßen des Menschen können sich Anomalien wie beispielsweise Wucherungen, Tumore oder Calziumablagerungen ausbilden, die nicht selten zum langsamen Zuwachsen und schließlich zum Verschluß des Gefäßes führen. Die Folge davon ist ein Infarkt, bei dem die Durchblutung und damit die Sauerstoffversorgung des entsprechenden Gewebes, beispielsweise des Herzens oder des Gehirns, unterbrochen ist.

Diese so gefährdeten Blutgefäße, beispielsweise Arterien, können über Katheter erreicht und mit Ballons und/oder Stents wieder aufgeweitet werden. Die Gewebeanomalien werden dabei verdrängt bzw. entfernt. Nun hat es sich gezeigt, daß bei Anwendung derartiger gefäßfremder medizinischer Hilfsmittel ein erneutes Zuwachsen (Re-Stenose) zu beobachten ist. Es ist bekannt, daß dieses Zuwachsen durch Isotopenbehandlung weitgehend beseitigt werden kann. Verwendet wird dazu eine Strahlenquelle, die mit einem Katheter in den Bereich des Stents geführt wird. Diese Strahlenquelle strahlt auf die an der Innenwandung des Blutgefäßes vorhandene Anomalie und durchdringt diese und die dahinter befindliche Gefäßwandung.

Ist in diesem Bereich jedoch ein Stent eingesetzt, der beispielsweise gitterförmig ausgebildet ist, so erreicht die Strahlung in den Bereichen, in denen Stent-Gittermaterial an der Gefäßinnenwandung bzw. an der Gefäßanomalie anliegt, zunächst das Stent-Material und die Gefäßanomalien bleiben quasi in dessen Schatten. Die in der Regel verwendeten β-Strahlen sind nicht energetisch genug, um das Stent-Material für eine Therapie tief genug zu durchdringen. Wird andererseits die β-Strahlenintensität erhöht, um die Stents zu durchdringen, so kann es zu Schädigungen der Gefäßinnenwandung kommen.

Es wurden deshalb Stents entwickelt, die die Radionuklide bereits beinhalten (EP 0 593 136 A1, WO 97/18012, DE 43 15 002 C1). Der Nachteil derartiger radioaktiver Gefäßimplantate besteht allerdings darin, daß sie nicht mehrfach verwendet werden können. Vor allem sind sie auch zur sogenannten "in stent therapy" nicht anwendbar, das heißt bei bereits vorhandenen Stents früherer erfolgloser Therapien.

Aufgabe der vorliegenden Erfindung war es deshalb, eine Strahlenquelle zu schaffen, die wiederverwendbar ist und zur "in stent therapy" eingesetzt werden kann, ohne daß es zu Schattenbildungen durch Stent-Gittermaterial kommt.

Gelöst wird die Aufgabe durch eine radioaktive Strahlenquelle, die ein Nuklid mit einer kombinierten β-γ-Strahlung mit 70-90% β-Strahlung und 30-10% γ-Strahlung beinhaltet. Diese Strahlenquelle gibt damit eine relativ breitbandige Strahlung ab. In den Bereichen, in denen Stent-Material an der Gefäßinnenwandung anliegt, wirkt die Strahlenquelle mit ihrem β-Anteil auf die Gefäßanomalie primär ein, während der vorhandene γ-Strahlenanteil zusätzlich durch das Stent-Material hindurch strahlt. Dadurch kann auch das Gewebe, welches quasi im Schatten von Fremdmaterial liegt, einer vorbestimmten Strahlendosis ausgesetzt werden. Der Anteil an γ-Strahlung von 10-30% hat sich als notwendig und ausreichend erwiesen, durch die Stentstreben eine notwendige Strahlendosis zu applizieren, ohne das nebenliegende Gewebe zu schädigen. Erfindungsgemäß können als Nuklide alle die eingesetzt werden, die eine kombinierte β-γ-Strahlung im genannten Verhältnis aussenden, bevorzugt wird Ruthenium -106 eingesetzt. Erfindungsgemäß ist es selbstverständlich auch möglich, daß die Strahlenquelle zwei Radionuklide enthält, wovon eins ein β-Strahler und das andere ein γ-Strahler ist. Dabei muß gewährleistet werden, daß von der γ-Strahlenquelle nur soviel vorhanden ist, daß 30-10% der Gesamtstrahlung auf die γ-Strahlung und 70-90% auf die 13-Strahlung entfallen. Für den Fachmann stellt es in der Regel kein Problem dar, die entsprechenden Mengen der einzusetzenden Nuklide zu ermitteln, damit das genannte Verhältnis gewahrt wird. Erfahrungsgemäß muß die Strahlenquelle dafür ca. 5 bis 10 mal mehr β-Strahlenträger als γ-Strahlenträger beinhalten. Erfindungsgemäß besonders bevorzugt ist ein Nuklidgemisch aus Strontium/Yttrium und Jod 125 oder Palladium 103.

Die Herstellung der erfindungsgemäßen radioaktiven Strahlenquelle erfolgt nach an sich bekannten Methoden, indem das/die Nuklid(e) in eine Trägermatrix eingebracht werden und diese Matrix in einem dünnwandigen Rohr aus körperverträglichem Material fixiert wird. Als Trägermaterial kommen alle üblichen Materialien in Frage, die in der Lage sind, die erfindungsgemäß verwendeten Nuklide aufzunehmen. Erfindungsgemäß bevorzugt werden Zeolithe oder andere Kunststoffe als Träger eingesetzt, aber auch Keramiken wie Titandioxid, Zirkoniumdioxid, Aluminiumoxid oder Siliziumoxid kommen in Frage. Das dünnwandige Rohr, in welches die Trägermatrix eingekapselt wird, besteht in einer bevorzugten Variante aus einer Metallegierung, z.B. einem Eisen und/oder Nickel enthaltenden Stahl oder einer Nickel/Titan-Legierung.

Gegenstand der Erfindung ist auch ein Verfahren zur Behandlung von Gefäßanomalien, indem die erfindungsgemäße Strahlenquelle mit einer kombinierten β-γ-Strahlung wie oben beschrieben in den zu behandelnden Gefäßbereich gebracht wird und die Gefäßanomalien mit Strahlen unterschiedlicher Eindringtiefe und Eindringintensität behandelt werden. Dazu wird die Strahlenquelle eine vorbestimmte Zeit, die zum Applizieren der effektiven Dosis notwendig ist, im zu behandelnden Gefäßbereich belassen. Das Einbringen der Strahlenquelle erfolgt üblicherweise mittels eines Katheters.

Die Erfindung ist in einer bevorzugten Ausführungsform in der beiliegenden Zeichnung dargestellt und wird nachfolgend näher beschrieben; es zeigt:
- Figur 1: die schematische Darstellung eines Gefäßes mit an der Gefäßinnenwandung abgesetzten Gefäßanomalien
- Figur 2: die schematische Darstellung eines mit Stents aufgeweiteten Gefäßes;
- Figur 3: die schematische Darstellung eines Gefäßes mit Stents und in den Bereich eingebrachter erfindungsgemäßer Strahlenquelle.

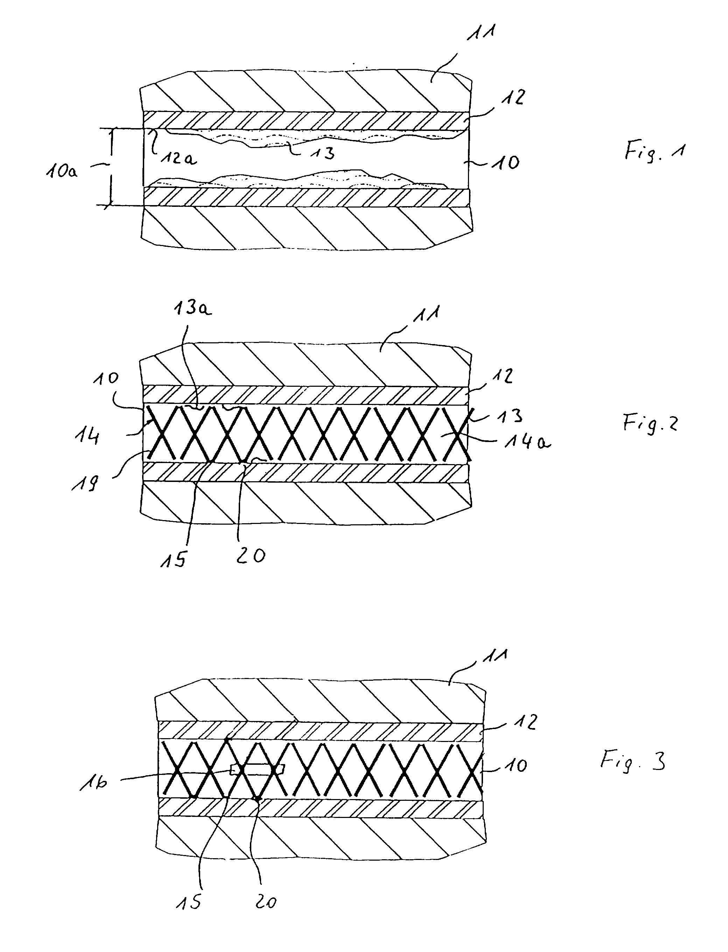

In der Figur 1 ist ein Blutgefäß 10 dargestellt, das von Gewebe 11 umgeben ist. An der Innenseite 12a der Gefäßwandung 12 haben sich Gefäßanomalien 13, beispielsweise Wucherungen, Tumore, Calziumablagerungen oder dgl. gebildet. Derartige Anomalien 13 bewirken eine Verengung der lichten Weite 10a des Gefäßes 10, die bis zum vollständigen Verschluß führen können. Durch derartige Gefäßverschlüsse wird die Blutzirkulation und damit die Sauerstoffversorgung des Gewebes unterbrochen und es kommt zum Infarkt.

Um das Gefäß 10 wieder für den lebensnotwendigen Bluttransport durchgängig zu machen, sind sogenannte Stents 14 bekannt, wie sie in der Figur 2 schematisch dargestellt sind. Diese Stents 14 können mit Hilfe von - nicht dargestellten - Kathetern in den Bereich der Anomalien 13 in das Gefäß 10 eingeführt werden.

In der vorgesehenen Position werden die Stents 14 dann mittels Druck von bis zu fünfzehn Bar aufgeweitet, bis sie die ursprüngliche lichte Weite 10a des Gefäßes 10 erreicht haben. Zugleich können die Anomalien 13 mit Hilfe von Isotopen zerstrahlt und damit retardiert werden. Die Stents 14 bleiben an ihrer Position und stützen das Gefäß 10 auch weiter ab.

Nun kommt es vor, daß sich neue Anomalien 13a bilden. Auch diese neuen Anomalien 13a sollen mit Isotopen therapiert und damit beseitigt werden. Es hat sich gezeigt, daß β-Strahlen durch die Stentstreben 19 zumindest teilweise absorbiert werden und die hinter den Anlagepunkten 15 der Stentstreben 19 - quasi in deren Schatten - vorhandenen Anomalien 13 nicht erreicht und zerstört werden.

Es kann dadurch zu erneutem Zuwachsen der Gefäße 10 und damit zu Beschwerden kommen. Nun kann die β-Strahlenintensität nicht beliebig erhöht werden, um die Anomalien 20 zu erreichen, die hinter den Anlagepunkten 15 der Stents 14 liegen. Würde die Menge an β-Strahlen über eine bestimmte Dosis hinaus erhöht werden, so würde das Gewebe 11 im Bereich der Zwischenräume 14a zwischen den Stentstreben 19 geschädigt werden.

In der Figur 3 ist die erfindungsgemäße Strahlenquelle 16 in Therapieposition dargestellt. Diese Strahlenquelle 16 enthält ein oder zwei Nuklide, die sowohl β-Strahlung als auch γ-Strahlung im erfindungsgemäßen Verhältnis aussenden. Da die γ-Strahlen härter sind, können sie die Stentstreben 19 durchdringen und die Anomalien 20, die im Schatten der Stentstreben 19 liegen, erreichen.

Die weichen β-Strahlen dieser breitbandigen Strahlenquelle 16 erreichen die Gefäßinnenseite 12a im Bereich der Zwischenräume 14a zwischen den Stentstreben 19, während die härteren γ-Strahlen durch die Stentanlagepunkte 15 hindurchgehen und die sich dahinter befindlichen Anomalien 20 zerstrahlen können.

Auf diese Weise kann die gesamte Gefäßinnenwandung 12a - auch im Bereich der eingesetzten Stents 14 - von Anomalien 13 bzw. 20 befreit und das Gefäß 10 wieder voll durchgängig gemacht werden.

Die erfindungsgemäße Strahlenquelle hat den Vorteil, daß sie im Gegensatz zu radioaktiven Stents wiederverwendbar und flexibler einsetzbar ist, da sie sowohl allein zur Behandlung von Gefäßanomalien eingesetzt werden kann als auch zusammen mit einem Stent. Die Strahlenquelle kann im Gegensatz zu radioaktiven Stents leicht und schnell in die Gefäße eingebracht oder herausgezogen werden. Durch das erfindungsgemäße Verhältnis von β- und γ-Strahlung löst die Strahlenquelle das spezifische Problem der Schattenbildung durch Stentmaterial.

### Bezugszeichenliste

- 10: Blutgefäß
- 10a: lichte Gefäßweite
- 11: Gewebe
- 12: Gefäßwandung
- 12a: Innenseite
- 13: Gefäßanomalie
- 13a: Gefäßanomalie
- 14: Stent
- 14a: Zwischenraum
- 15: Anlagepunkt
- 16: Strahlenquelle
- 19: Stentstrebe
- 20: Anomalie

## Patentansprüche

1. Radioaktive Strahlenquelle zur Behandlung von Gefäßanomalien,
dadurch gekennzeichnet, daß
die Strahlenquelle ein Nuklid mit einer kombinierten β-γ-Strahlung mit 70-90% β-Strahlung und 30-10% γ-Strahlung beinhaltet.

2. Strahlenquelle nach Anspruch 1,
dadurch gekennzeichnet, daß
das Nuklid Ruthenium-106 ist.

3. Verfahren zur Behandlung von Gefäßanomalien mittels radioaktiver Strahlung,
dadurch gekennzeichnet, daß
eine radioaktive Strahlenquelle, die ein Nuklid mit einer kombinierten β-γ-Strahlung mit 70-90% β-Strahlung und 30-10% γ-Strahlung beinhaltet, in den zu behandelnden Gefäßbereich gebracht wird und dort eine vorbestimmte Zeit, die zum Applizieren der effektiven Dosis notwendig ist, belassen wird.

4. Verfahren nach Anspruch 3,
dadurch gekennzeichnet, daß
eine radioaktive Strahlenquelle verwendet wird, die Ruthenium-106 als kombinierten β-γ-Strahler beinhaltet.

5. Verwendung einer radioaktiven Strahlenquelle, die ein Nuklid mit einer kombinierten β-γ-Strahlung mit 70-90% β-Strahlung und 30-10% γ-Strahlung beinhaltet, zur Behandlung von Gefäßanomalien.

6. Verwendung nach Anspruch 5,
dadurch gekennzeichnet, daß
die radioaktive Strahlenquelle zusammen mit einem Stent zur Behandlung von Gefäßanomalien eingesetzt wird.

7. Verwendung nach Anspruch 5 oder 6,
dadurch gekennzeichnet, daß
als Nuklid Ruthenium-106 eingesetzt wird.
